# EUROPEAN PATENT SPECIFICATION

(11) **EP 0 781 135 B1**
(45) Date of publication and mention of the grant of the patent: **04.12.2002**
(21) Application number: 95931856.9
(22) Date of filing: 14.09.1995
(51) Int. Cl.: A61K 31/485, A61K 47/48, A61K 9/20

(54) **CODEINE CONTAINING FORMULATIONS**
CODEIN ENTHALTENDE FORMULIERUNGEN
FORMULATIONS CONTENANT DE LA CODEINE

(30) Priority: 14.09.1994 AU PM814994
(43) Date of publication of application: 02.07.1997
(73) Proprietor: SmithKline Beecham plc, Brentford, Middlesex TW8 9GS (GB)
(72) Inventor: CHAN, Shing Yue, Cherrybrook, NSW 2126 (AU); JAMES, David Charles, West Ryde, NSW 2114 (AU); OWEN, Peter, Berowra, NSW 2031 (AU)
(74) Representative: White, Susan Mary
(86) International application number: AU9500601
(87) International publication number: WO96008252

(56) References cited:
- EP-A- 0 274 845
- US-A- 4 070 494
- US-A- 4 971 785

## Description

### BACKGROUND OF THE INVENTION

The present invention relates to the use of an effective amount of an anionic surface active agent in the manufacture of a solid pharmaceutical dosage form containing an analgesically active amount of codeine to hinder extraction of codeine from the dosage form by filtration and/or solvent extraction of the filtrate without preventing the release of codeine in the human digestive system.

Codeine is a morphine derivative which is commonly used in strong analgesic products. Lower dose codeine products, particularly those which combine codeine and paracetamol (acetaminophen), are readily available without prescription in many countries and are extremely useful tools in the treatment of strong pain.

It is possible to extract the codeine from analgesic products and convert the codeine to morphine and heroin. Although in most situations it is not economically viable to do this, it can become viable when the supply of illegal heroin is restricted and prices rise.

The process which has been devised to extract codeine from legally obtained products and convert this into heroin is known colloquially as the "homebake" process. The details of that process will not be disclosed here, except to say that the initial physical separation processes for extracting the codeine from tablets include the processes of slurrying, filtration and solvent extraction.

### SUMMARY OF THE INVENTION

The use according to the present invention aims to provide a solid pharmaceutical dosage form, and in particular a tablet formulation, which makes the homebake process more difficult without detracting from the effectiveness of the product when used as directed.

The invention is characterised by the inclusion in a codeine-containing pharmaceutical formulation of excipients which interfere with the homebake process. Preferably, these excipients interfere with the physical filtration and/or liquid extraction steps of the process rather than the subsequent chemical steps.

### DETAILED DESCRIPTION OF THE INVENTION

The use according to the invention provides a solid pharmaceutical dosage form containing an analgesically active amount of codeine, said dosage form further including an amount of a constituent effective to hinder extraction of codeine from the dosage form by filtration and/or solvent extraction of the filtrate without preventing the release of codeine in the human digestive system, said constituent being selected from:
(a) from 1-10% by weight of the total dosage form of a film forming agent,
(b) an anionic surface active agent, and
(c) an ion exchange resin.

Preferred film forming agents for use in the invention are the cellulose derivatives and especially hydroxypropylmethyl cellulose, methylcellulose and hydroxypropylcellulose. The film formers should be included in an amount which is sufficient to impair extraction by the 'homebake' process but insufficient to cause the dosage form to display slow release characteristics in the human digestive system. Thus amounts less than 10% should be included in the formulation, preferably 1-10% and most preferably 1-5% by weight of the total dosage form.

Preferred anionic surface active agents are sodium lauryl sulfate, sodium lauryl ether sulfate and any of the alkylaryl sulfonates. Sodium lauryl sulfate has been found by the inventors to be highly effective for this purpose and is particularly preferred. The anionic surface active agents are preferably included in an amount from 0.1-3% by weight of the dosage form.

Preferred ion exchange resins are polacrilin potassium and pharmaceutically acceptable acid cation exchange resins such as sulfonic acid cation exchange resins or carboxylic acid cation exchange resins. The ion exchange resin is preferably included in an amount from 2-15% by weight of the dosage form.

Formulations containing codeine as the only active, or codeine and paracetamol only, are preferred.

Further preferred embodiments will be apparent from the Examples, which describe methods of formulating combined codeine and paracetamol tablets with improved resistance to illicit codeine extraction by the homebake process.

Each of the exemplary formulations has a similar base formulation to which the anti-homebake ingredient is added. The tablets may then further be film coated with a coating including a cellulose derivative film forming agent, the amount of coating applied effecting a weight increase of between about 2% and 5% of the tablet. However, it is preferred that tablets incorporating anionic surfactant not be film coated as preliminary testing suggests that this combination may in fact be less effective than anionic surfactant alone.

The base formulation is as follows:
- Actives -: Paracetamol 500mg/tab and Codeine Phosphate 8mg/tab.
- Binder -: For example, Povidone K25 3-8% w/w or Maize Starch 5-15% w/w.
- Disintegrant -: For example, Sodium Starch Glycolate 3-8% w/w
- Lubricant -: For example, Magnesium Stearate at 0.5-1.5% or Stearic Acid 1-3%.
- Flow aid -: For example, Colloidal Silica 0.2-1% w/w or Talc 1-3%.
- Diluents -: For example, starch or lactose remainder as a bulking agent.

### Example 1

The desired amount of codeine phosphate and anti-homebaking ingredient - an anionic surfactant or cellulose derivative - is blended dry into pre-granulated paracetamol. The granulation is then pressed into tablets using conventional tablet pressing machinery. if the anti-homebake ingredient is a cellulose derivative, the pressed tablets are film coated with a coating including a cellulose film forming agent, to a 5% weight increase.

### Example 2

The actives, binder, granule disintegrant and anti-homebake ingredient - an anionic surfactant or a cellulose film forming agent - are sieved and added to a high shear mixer/granulator. After pre-mixing, the materials are granulated then partially dried in a fluid bed dryer. The partially dried product is sieved and returned to the fluid bed dryer for final drying. The dried granules are blended with the lubricant and the flow aid (if required) and then pressed into tablets. If the anti-homebake ingredient is a cellulose derivative, the tablets are then film coated with a cellulose film forming agent to a 5% weight increase.

### Example 3

Codeine (either as base or phosphate) is dissolved in a suitable solvent and passed through a bed of cation exchange resin, which may be in either the acid form or as the sodium salt, depending on the acid strength of the resin. The codeine solution can be recycled through the exchange resin if necessary in order to ensure sufficient binding of the codeine to the exchange resin.

The resulting codeine resinate is dried and is sieved and milled if necessary. The resultant powder can be granulated if required (some products are quite granular and have suitable properties for tablet pressing without further processing).

The granular resinate, together with the binder and disintegrant, is then dried in a fluid bed, sieved through an oscillating granulator and then blended with the lubricants and flow aids. The final granulation is pressed into tablets, which are then film coated to between 2% to 5% weight increase.

With legitimate use of the product the anti-homebake ingredient is designed to release from the tablet and have no effect on the action or release rate of codeine. However, in vitro, the ingredients have specific properties which enable it to confound certain steps in the process of illicit codeine extraction.

The inclusion of additives in codeine-containing tablets, according to the invention, interferes with the homebake process by increasing the processing time and/or decreasing extraction efficiency. In particular, the additives may increase the time taken to filter the slurry and/or may emulsify the two phases in the liquid-liquid extraction step so that the separation of the two layers is inefficient.

In the case of a cellulosic film former or anionic surfactant, the ingredient chosen should be sufficiently water soluble not to be left behind in the solid phase as the codeine is dissolved when the crushed tablets are slurried up. The film formers interfere with the homebaking process by forming a gel when contacted with water, binding up the water phase and clogging up the filtration and also by inhibiting the solvent extraction of the codeine. The anionic surfactants work by emulsifying the solvent and liquid phases in the solvent extraction. The ion exchange resins are generally insoluble and lock up the codeine so that it cannot be extracted.

The inclusion of 2.5% by weight of hydroxymethylpropyl -cellulose in the tablet formulation according to the invention has been found to reduce the extraction of codeine by approximately 45-65%. Sodium lauryl sulphate has been found to reduce the extraction by approximately 25% when incorporated at 0.9% by weight, and by greater than 90% when incorporated at 2.5% by weight. Of the ion exchange resins tested, Zeolex 23A and Amberlite IRP69 (a styrene based sulfonic acid cation exchange resin) gave very good results, both reducing codeine extraction by approximately 60%.

It is strongly preferred that the anti-homebake ingredient be distributed throughout the matrix of the tablet, to reduce the possibility of physical separation of the ingredient prior to the homebake process.

## Claims

1. The use of an effective amount of an anionic surface active agent in the manufacture of a solid pharmaceutical dosage form containing an analgesically active amount of codeine, to hinder extraction of codeine from the dosage form by filtration and/or solvent extraction of the filtrate without preventing the release of codeine in the human digestive system.

2. The use according to claim 1 wherein said dosage form is a tablet.

3. The use according to claim 2 wherein said anionic surface active agent is distributed throughout the tablet.

4. The use according to any of claims 1 to 3 wherein said anionic surface active agent is present in an amount of 0.1 to 3% by weight of the dosage form.

5. The use according to claim 4 wherein said anionic surface active agent is selected from sodium lauryl sulfate, sodium lauryl ether sulfate or an alkylaryl sulfonate.

6. The use according to claim 5 wherein said anionic surface active agent is sodium lauryl sulfate.

## Patentansprüche

1. Verwendung einer wirksamen Menge eines anionischen Tensids in der Herstellung einer festen pharmazeutischen Arzneiform, die eine analgetisch wirksame Menge Codein enthält, um die Extraktion von Codein aus der Arzneiform durch Filtration und/oder Lösungsmittelextraktion des Filtrats zu behindern, ohne die Freisetzung von Codein im menschlichen Verdauungssystem zu unterbinden.

2. Verwendung gemäß Anspruch 1, worin die Arzneiform eine Tablette ist.

3. Verwendung gemäß Anspruch 2, worin das anionische Tensid in der Tablette verteilt ist.

4. Verwendung gemäß einem der Ansprüche 1 bis 3, worin das anionische Tensid in einer Menge von 0,1 bis 3 % des Gewichts der Arzneiform vorhanden ist.

5. Verwendung gemäß Anspruch 4, worin das anionische Tensid aus Natriumlaurylsulfat, Natriumlaurylethersulfat oder einem Alkylarylsulfonat ausgewählt ist.

6. Verwendung gemäß Anspruch 5, worin das anionische Tensid Natriumlaurylsulfat ist.

## Revendications

1. Utilisation d'une quantité efficace d'un agent tensioactif anionique dans la fabrication d'une forme galénique pharmaceutique solide contenant une quantité de codéine active du point de vue analgésique pour empêcher l'extraction de codéine de la forme galénique par filtration et/ou extraction par solvant du filtrat, sans empêcher la libération de la codéine dans le système digestif humain.

2. Utilisation suivant la revendication 1, dans laquelle ladite forme galénique est un comprimé.

3. Utilisation suivant la revendication 2, dans laquelle ledit agent tensioactif anionique est distribué dans la totalité du comprimé.

4. Utilisation suivant l'une quelconque des revendications 1 à 3, dans laquelle ledit agent tensioactif anionique est présent en une quantité de 0,1 à 3% en poids de la forme galénique.

5. Utilisation suivant la revendication 4, dans laquelle ledit agent tensioactif anionique est choisi parmi le lauryl sulfate de sodium, le lauryl éther sulfate de sodium ou un alkylaryl sulfonate.

6. Utilisation suivant la revendication 5, dans laquelle ledit agent tensioactif anionique est le lauryl sulfate de sodium.
